# EUROPEAN PATENT APPLICATION

(11) **EP 4 302 814 A1**
(43) Date of publication of application: **10.01.2024**
(21) Application number: 22762871.6
(22) Date of filing: 01.02.2022
(51) Int. Cl.: A61M 25/09

(54) **GUIDE WIRE**

(30) Priority: 03.03.2021 JP 2021033173
(71) Applicant: Asahi Intecc Co., Ltd., Seto-shi, Aichi 489-0071 (JP)
(72) Inventor: USHIDA, Keisuke, Seto-shi, Aichi 489-0071 (JP)
(74) Representative: Prinz & Partner mbB
(86) International application number: PCT/JP2022/003746
(87) International publication number: WO 2022/185813

(57) **Abstract**

A guide wire having a good flexibility on a distal end portion, while ensuring a joining strength between a coil body and a core shaft is provided. The guide wire includes a core shaft, a coil body that covers an outer periphery of the core shaft, and a distal end side joint part that joins a distal end portion of the coil body with a distal end portion of the core shaft. The distal end side joint part is made of a resin material. The guidewire further includes a fixation part made of a metal material that joins the coil body with the core shaft on a proximal end side of the distal end side joint part.

## Description

### TECHNICAL FIELD

The technology disclosed herein relates to a guide wire.

### BACKGROUND ART

In general, guide wires or the like that are inserted into a body, e.g. into a blood vessel, require good insertability into the blood vessel or the like, and good operability in the blood vessel or the like. In general, a guide wire includes a core shaft, a coil body that covers an outer periphery of the core shaft, and a distal end side joint part that joins a distal end portion of the coil body with a distal end portion of the core shaft.

As for such a guide wire, a guide wire in which the distal end portion of the coil body is fixed to the core shaft by an Au-Sn solder is known (e.g. see Patent Literature 1).

### CITATION LIST

### Patent Literature

Patent Literature 1: JP 2010-214054 A

### SUMMARY OF INVENTION

### TECHNICAL PROBLEM

As mentioned above, guide wires that are inserted into a blood vessel or the like require a strong joint between the coil body and the core shaft, and also require good insertability and operability. For example, when a guide wire is advanced from a main vessel with a large vessel diameter to a bifurcated vessel with a small vessel diameter, the distal end of the guide wire may abut against an inner wall of the vessel at an entrance of the bifurcated vessel. In such a case, there is a tendency that, if the distal end portion of the guide wire has low flexibility (stiff), resistance on the distal end portion of the guide wire increases when the guide wire is further pushed forward, making it more difficult to advance the guide wire toward the distal end side of the bifurcated vessel.

These problems are not limited to the case that the guide wire is advanced from the main vessel to the bifurcated vessel, but are also common to cases in which the guide wire is advanced in a vessel having a complex shape.

The present specification discloses techniques capable of solving the problems described above.

### SOLUTION TO PROBLEM

The technology disclosed herein can be implemented, for example, as the following aspects.
(1) The guide wire disclosed herein includes a core shaft, a coil body that covers an outer periphery of the core shaft, and a distal end side joint part that joins a distal end portion of the coil body with a distal end portion of the core shaft. The distal end side joint part is made of a resin material. The guide wire further includes a fixation part made of a metal material that joins the coil body with the core shaft on a proximal end side of the distal end side joint part. In the guide wire according to the disclosed embodiments, since the distal end side joint part is made of the resin material, the slidability and flexibility on the distal end portion of the guide wire can be improved. In the guide wire according to the present invention, since the fixation part is made of the metal material, the coil body and the core shaft can be firmly joined. Thus, the guide wire according to the present invention makes it possible to obtain a good flexibility on the distal end portion, while ensuring a joining strength between the coil body and the core shaft.
(2) The guide wire may be configured such that the distal end side joint part is made of an elastomer material. According to the guide wire having this configuration, flexibility on the distal end portion of the guide wire can be enhanced, and therefore, when the guide wire is inserted into a blood vessel or the like, the inner wall of the blood or the like can be more reliably prevented from being damaged.
(3) The guide wire may be configured such that a gap between the distal end side joint part and the fixation part in the axial direction of the guide wire is smaller than a length of the distal end side joint part in the axial direction. Thus, in the guide wire having this configuration, the gap between the distal end side joint part and the fixation part is relatively small. In other words, in this configuration, the fixation part is located in the vicinity of the distal end of the guide wire. Thus, in the guide wire according to the present invention, the joining strength between the coil body and the core shaft can be more effectively ensured on the distal end of the guide wire.
(4) The guide wire may be configured such that the distal end side joint part and the fixation part are continuously arranged in an axial direction of the guide wire. In other words, in the guide wire having this configuration, the fixation part is located in the vicinity of the distal end of the guide wire. Thus, in the guide wire according to the present invention, the joining strength between the coil body and the core shaft can be more effectively ensured on the distal end of the guide wire.
(5) The guide wire may be configured such that a length of the distal end side joint part in an axial direction of the guide wire is twice or larger and 10 times or smaller an outer diameter of the coil body. Thus, according to the guide wire having this configuration, the joining strength between the distal end side joint part and the coil body can be more effectively ensured.
(6) The guide wire may be configured so as to include a resin film that covers an outer surface of the distal end side joint part and an outer peripheral surface of the coil body and is made of one or a plurality of resin materials, in which the resin material for the distal end side joint part is the same as at least one of the resin materials for the resin film. Thus, according to the guide wire having this configuration, the adhesiveness between the distal end side joint part and the resin film can be improved, and therefore the resin film can be prevented from peeling off from the distal end side joint part.

The technology disclosed herein can be achieved in various aspects, e.g. in an aspect of a guide wire or a production method thereof.

### BRIEF DESCRIPTION OF DRAWINGS

FIG. 1 is a schematic explanatory diagram illustrating a longitudinal sectional configuration of a guide wire 100 according to a first embodiment.
FIG. 2 is a partial enlarged view of the guide wire 100.
FIG. 3 is a schematic explanatory diagram illustrating a longitudinal sectional configuration of a guide wire 100A according to a second embodiment.
FIG. 4 is a schematic explanatory diagram illustrating a longitudinal sectional configuration of a guide wire 100B according to a third embodiment.

### EMBODIMENTS OF THE INVENTION

### A. First Embodiment:

### A-1. Configuration of Guide Wire 100:

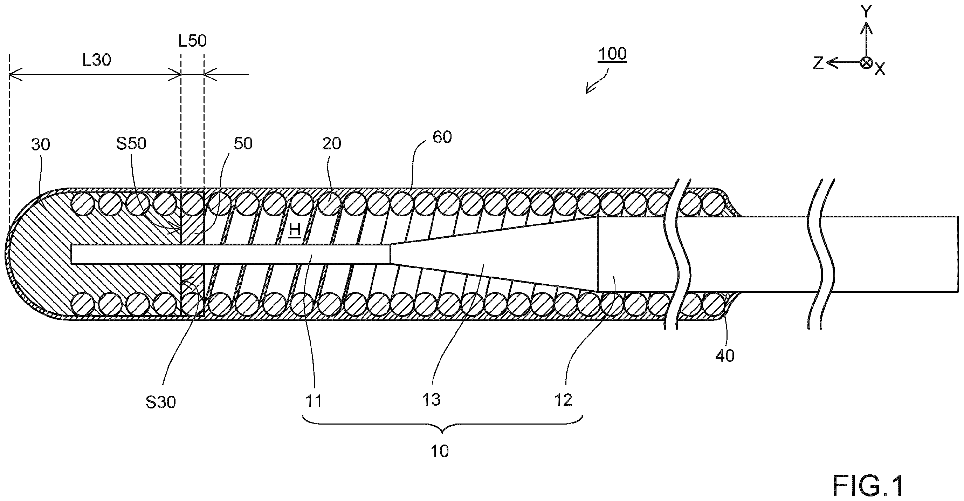

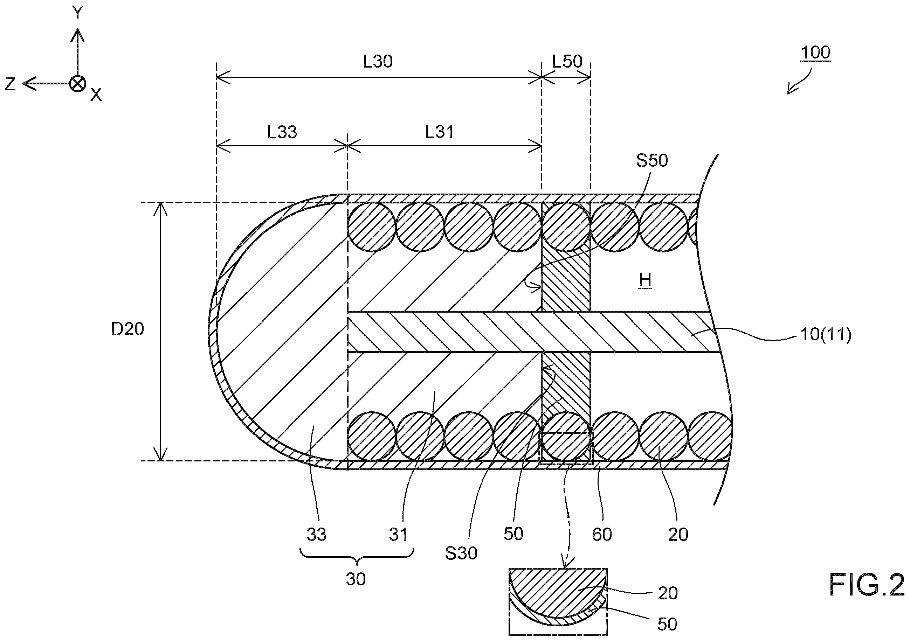

FIG. 1 is a schematic explanatory diagram illustrating a configuration of a guide wire 100 according to a first embodiment. FIG. 1 illustrates a longitudinal section (YZ section) of the guide wire 100. In FIG. 1, a positive direction side in Z-axis is a distal end side (far side) to be inserted into the body, and a negative direction side in Z-axis is a proximal end side (near side) to be manipulated by a technician such as a surgeon. The same applies to FIG. 2 and subsequent figures. FIG. 1 illustrates sectional configurations (specifically, YZ section) of a coil body 20, a distal end side joint part 30, a proximal end side joint part 40, a metal fixation part 50, and a resin part 60, which are described later. Although FIG. 1 illustrates a state in which the guide wire 100 is wholly in a straight-line shape substantially parallel to the Z-axis direction, the guide wire 100 is flexible enough to be curved. In the guide wire 100 and each component of the guide wire 100, a part that includes the distal end and extends toward the proximal end side from the distal end to the middle is hereinafter referred to as a "distal end portion". Similarly, in the guide wire 100 and each component of the guide wire 100, a part that includes the proximal end and extends toward the distal end side from the proximal end to the middle is referred to as a "proximal end portion".

The guide wire 100 is a medical device to be inserted into a blood vessel or the like, in order to guide a catheter (not illustrated) into e.g. a lesion (constricted part or occluded part) in a blood vessel or the like.

As illustrated in FIG. 1, the guide wire 100 includes a core shaft 10, the coil body 20, the distal end side joint part 30, the proximal end side joint part 40, the metal fixation part 50, and the resin part 60. The metal fixation part 50 is an example of a fixation part.

The core shaft 10 is an elongated member having a small diameter on the distal end side and a large diameter on the proximal end side. In the first embodiment, the core shaft 10 has a small diameter portion 11, a large diameter portion 12, and a tapered portion 13. In FIG. 1, illustration of the large diameter portion 12 is partially omitted. The small diameter portion 11 includes the distal end of the core shaft 10. The small diameter portion 11 has a rod shape with a circular transverse section. The transverse section refers to a section (XY section in the first embodiment) perpendicular to an axial direction (Z-axis direction in the first embodiment) of the core shaft 10 (the same applies to the second embodiment and following embodiments). In the first embodiment, the axial direction of the core shaft 10 coincides with the axial direction of the guide wire 100. The large diameter portion 12 is located on the proximal end side of the core shaft 10 relative to the small diameter portion 11 and has a rod shape having a circular transverse section with a larger outer diameter than of the small diameter portion 11. The tapered portion 13 is located between the small diameter portion 11 and the large diameter portion 12. The tapered portion 13 gradually increases in the outer diameter from the boundary position with the small diameter portion 11 to the boundary position with the large diameter portion 12. For example, the small diameter portion 11 has an outer diameter of about 0.03 to 0.085 mm, and the large diameter portion 12 has an outer diameter of about 0.2 to 0.9 mm. A transverse-sectional shape of each portion of the core shaft 10 is not particularly limited, and may be polygonal e.g. triangular or quadrangular.

As the material for the core shaft 10, known materials are used, for example, metallic materials, more specifically, stainless steel (SUS302, SUS304, SUS316, etc.), superelastic alloys such as Ni-Ti alloys, piano wires, nickel-chromium based alloys, cobalt alloys, tungsten or the like are used. The core shaft 10 may be made entirely of one material, or each portion of the core shaft 10 may be made of different materials.

As illustrated in FIG. 1, the coil body 20 is a coil-shaped member formed into a hollow-cylindrical shape by spirally winding one wire. In the first embodiment, the coil body 20 is arranged so as to surround the outer periphery of the distal end portion of the core shaft 10 (specifically, the small diameter portion 11, the tapered portion 13, and a part of the large diameter portion 12). A lumen H is formed between the core shaft 10 and the coil body 20. For example, the coil body 20 has a total length of about 10 mm to 500 mm and an outer diameter D20 (see FIG. 2) of about 0.2 mm to 0.9 mm.

As the material for forming the coil body 20, known materials are used, for example, metallic materials, more specifically, stainless steel (SUS302, SUS304, SUS316, etc.), superelastic alloys such as Ni-Ti alloys, piano wires, nickel-chromium based alloys, cobalt alloys, tungsten or the like are used.

The distal end side joint part 30 joins the distal end portion of the core shaft 10 with the distal end portion of the coil body 20. The distal end of the core shaft 10 and the distal end of the coil body 20 are fixed so as to be embedded in the distal end side joint part 30. The distal end side joint part 30 has, on the distal end side, a smooth outer peripheral surface (e.g., a substantially hemispherical surface and a cylindrical surface). Preferably, the distal end side joint part 30 is elastic and flexible and is made of resin materials. For such resin materials, e.g. elastomer materials are used, such as thermosetting elastomer resins and thermoplastic elastomer resins. As thermosetting elastomer resins, for example, silicone rubbers, fluororubbers or the like are used. As thermoplastic elastomers, for example, polyurethane resins, polyester resins, polyamide resins or the like are used. More preferably, the distal end side joint part 30 contains at least one of the resin materials for the resin part 60 described later. For such resins, e.g. hydrophilic resins or the like, more preferably polyurethane resins or the like are used. The distal end side joint part 30 may be made of one or a plurality of resin materials. When the distal end side joint part 30 having flexibility on the distal end side is disposed on the core shaft 10, a blood vessel wall can be prevented from being damaged. Also, the resin part 60 makes it possible to improve the slidability of the distal end portion of the guide wire 100. The detailed configuration of the distal end side joint part 30 will be described later.

The proximal end side joint part 40 is a member that joins the proximal end side of the core shaft 10 with the proximal end side of the coil body 20. As the materials constituting the proximal end side joint part 40, known materials are used, such as brazing materials (aluminum alloy braze, silver braze, gold braze, etc.), metal solders (Ag-Sn alloys, Au-Sn alloys, etc.), and adhesives (epoxy-based adhesives, etc.). In the first embodiment, brazing materials are used as the materials constituting the proximal end side joint part 40.

The metal fixation part 50 is a member that joins the coil body 20 with the core shaft 10 on the proximal end side of the distal end side joint part 30. In the first embodiment, the core shaft 10 and the coil body 20 are joined to each other such that a part of each of the core shaft 10 and the coil body 20 is embedded in the metal fixation part 50. More specifically, in the first embodiment, the metal fixation part 50 is formed so as to cover the outer surface of a part of the coil body 20 described above (i.e., a part where the metal fixation part 50 is disposed) such that a thickness of the metal fixation part 50 is substantially uniform, as illustrated in the partial enlarged view of FIG. 2. As the materials for the metal fixation part 50, metal materials are used, such as metal solders (Au-Sn alloy, Sn-Ag alloy, Sn-Pb alloy, Pb-Ag alloy, etc.), and brazing materials (aluminum alloy braze, silver braze, gold braze, etc.). The materials for the metal fixation part 50 and the proximal end side joint part 40 may be the same as or different from each other. The detailed configuration of the metal fixation part 50 will be described later.

The resin part 60 is a coat member that is made of one or a plurality of resin materials and covers the outer peripheral surface of the coil body 20 and the outer surfaces of the distal end side joint part 30 and the proximal end side joint part 40. As the resin materials constituting the resin part 60, known materials are used, such as silicone resins. As the resin materials constituting the resin part 60, hydrophilic resins are preferably used, and hyaluronic acid-based resins, polyvinyl alcohol (PVA) resins or the like are more preferably used. For example, the resin part 60 has a thickness of about 0.002 to 0.1 mm. The resin part 60 is an example of a resin film.

A-2. Detailed Configuration of the distal end side joint part 30 and the metal fixation part 50:
FIG. 2 is an explanatory diagram illustrating the configuration of the distal end side joint part 30 and the metal fixation part 50, in which a longitudinal section (YZ section) of the configuration is enlarged.

The distal end side joint part 30 has a fixing portion 31 and a most distal end portion 33. The fixing portion 31 is located on the most proximal end side of the distal end side joint part 30 to fix the distal end portion of the coil body 20 with the distal end portion of the core shaft 10. The most distal end portion 33 is disposed on the distal end of the fixing portion 31 in the distal end side joint part 30 and has an almost spherical segment shape with an outer diameter gradually decreasing toward the distal end side. The outer diameter on the distal end of the fixing portion 31 is equal to the outer diameter on the proximal end of the most distal end portion 33. That means, the outer surface of the fixing portion 31 is continuous with the outer surface of the most distal end portion 33. In the first embodiment, the fixing portion 31 and the most distal end portion 33 have an almost circular transverse sectional shape (XY section).

For example, the distal end side joint part 30 has a length L30 of about 0.5 mm to 5 mm in the Z-axis direction (axial direction of the core shaft 10). The length L30 of the distal end side joint part 30 may be e.g. about twice or larger and 10 times or smaller, more specifically about 3 times an outer diameter D20 of the coil body 20. For example, the most distal end portion 33 has a length L33 of about 0.45 mm to 1 mm. In the first embodiment, the length L30 of the distal end side joint part 30 is about 4 mm. Preferably, at least four turns of the coil body 20 are embedded in the distal end side joint part 30 in the longitudinal section.

In the Z-axis direction, a length L50 of the metal fixation part 50 is e.g. about 0.5 mm to 1.5 mm, more specifically about 0.5 mm to 1 mm, from the viewpoint of ensuring the joining strength between the coil body 20 and the core shaft 10. For example, the metal fixation part 50 is located at most 5 mm apart from the most distal end of the guide wire 100, from the viewpoint of ensuring the joining strength between the coil body 20 and the core shaft 10 on the distal end of the guide wire 100.

In the guide wire 100 according to the first embodiment, an end face S30 on the proximal end side of the distal end side joint part 30 (specifically, fixing portion 31) and an end face S50 on the distal end side of the metal fixation part 50 are in contact with each other over the entire face. In other words, in the Z-axis direction, the distal end side joint part 30 and the metal fixation part 50 are continuously arranged. In FIG. 1 and FIG. 2, for convenience, the end face S30 of the distal end side joint part 30 and the end face S50 of the metal fixation part 50 are each depicted as a surface perpendicular to the Z-axis direction, but the present invention is not limited to this configuration. The same applies to the following figures.

### A-3. Production Method for Guide Wire 100:

For example, the guide wire 100 according to the first embodiment can be produced by a method described below. First, the core shaft 10 shaped by machine polishing or the like, and the coil body 20 composed of a wound coil wire are prepared. The core shaft 10 is inserted into a hollow portion of the coil body 20, and then the distal end side joint part 30, the proximal end side joint part 40, and the metal fixation part 50, which join the coil body 20 and core shaft 10 are formed. To form the distal end side joint part 30, for example, a molten resin is injected into a mold capable of shaping the distal end side joint part 30, the distal end portions of the core shaft 10 and coil body 20 are dipped into the resin, followed by cooling. Then, to form the metal fixation part 50, a molten brazing material is injected from a gap of the coil body 20 into a position at which the metal fixation part 50 is formed, followed by cooling. The proximal end side joint part 40 is formed by brazing the proximal end side of the coil body 20 with the core shaft 10. Subsequently, these members are dipped into a molten resin for forming the resin part 60 to form the resin part 60. The guide wire 100 having the aforementioned configuration can be produced e.g. by the method as described above.

### A-4. Effects of Embodiment:

As described above, the guide wire 100 according to the first embodiment includes the core shaft 10, the coil body 20, and the distal end side joint part 30. The distal end side joint part 30 is made of a resin material. The guide wire 100 further includes a metal fixation part 50. The metal fixation part 50 joins the coil body 20 with the core shaft 10 on the proximal end side of the distal end side joint part 30 and is made of a metal material. In the guide wire 100 according to the first embodiment, since the distal end side joint part 30 is made of a resin material, the slidability and flexibility on the distal end portion of the guide wire 100 can be improved. In the guide wire 100 according to the first embodiment, since the metal fixation part 50 is made of a metal material, the coil body 20 and the core shaft 10 can be firmly joined. Thus, the guide wire 100 according to the first embodiment makes it possible to obtain a good flexibility on the distal end portion, while ensuring the joining strength between the coil body 20 and the core shaft 10.

In the guide wire 100 according to the first embodiment, the distal end side joint part 30 is made of an elastomer material. In the guide wire 100 according to the first embodiment, flexibility of the distal end portion of the guide wire 100 can be enhanced, and therefore, when the guide wire is inserted into a blood vessel or the like, a blood vessel inner wall or the like can be more reliably prevented from being damaged.

In the guide wire 100 according to the first embodiment, the distal end side joint part 30 and the metal fixation part 50 are continuously arranged in the axial direction of the guide wire 100. In other words, in the guide wire according to the first embodiment, the metal fixation part 50 is located in the vicinity of the distal end of the guide wire 100. Thus, in the guide wire 100 according to the first embodiment, the joining strength between the coil body 20 and the core shaft 10 can be more effectively ensured on the distal end of the guide wire 100.

In the guide wire 100 according to the first embodiment, a length of the distal end side joint part 30 in the axial direction of the guide wire 100 is twice or larger and 10 times or smaller an outer diameter D20 of the coil body 20. Thus, in the guide wire 100 according to the first embodiment, the joining strength between the distal end side joint part 30 and the coil body 20 can be more effectively ensured.

The guide wire 100 according to the first embodiment further includes the resin part 60, and the resin material for the distal end side joint part 30 is the same as at least one of the resin materials for the resin part 60. Thus, in the guide wire 100 according to the first embodiment, the adhesiveness between the distal end side joint part 30 and the resin part 60 can be improved, and therefore the resin part 60 can be prevented from peeling off from the distal end side joint part 30.

### B. Second Embodiment:

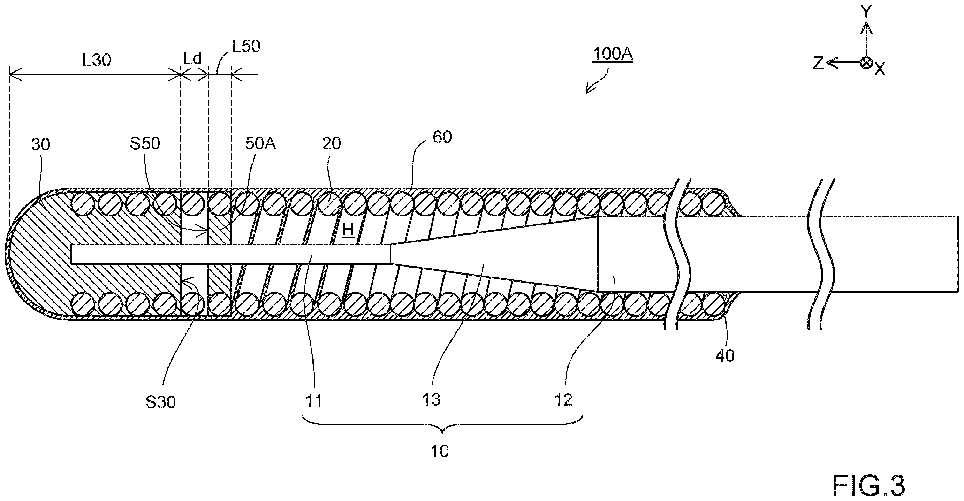

FIG. 3 is a schematic explanatory diagram illustrating a longitudinal sectional configuration (YZ section) of a guide wire 100A according to the second embodiment. Hereinafter, components of the guide wire 100A according to the second embodiment, which are identical to the components of the guide wire 100 according to the first embodiment, are provided with the same reference signs as in the first embodiment, thereby omitting the description thereof as appropriate.

The guide wire 100A according to the second embodiment includes a metal fixation part 50A instead of the metal fixation part 50 in the guide wire 100 according to the first embodiment. Similarly to the metal fixation part 50, the metal fixation part 50A is a member that joins the coil body 20 with the core shaft 10 on the proximal end side of the distal end side joint part 30 and is made of the metal materials described above. In the second embodiment, the metal fixation part 50A is located away from the distal end side joint part 30. That means, the end face S30 of the distal end side joint part 30 and the end face S50 of the metal fixation part 50A are not in contact with each other over the entire face. More specifically, a gap Ld between the distal end side joint part 30 and the metal fixation part 50A is smaller than the length L30 of the distal end side joint part 30, e.g. about 0.5 mm to 1 mm. The length L50 of the metal fixation part 50A is equivalent to the length L50 of the metal fixation part 50, e.g. about 0.5 mm to 1.5 mm, more specifically, about 0.5 mm to 1 mm. For example, the metal fixation part 50A is located at most 5 mm apart from the most distal end of the guide wire 100, from the viewpoint of ensuring the joining strength between the coil body 20 and the core shaft 10 on the distal end of the guide wire 100.

In the guide wire 100A according to the second embodiment, the gap Ld between the distal end side joint part 30 and the metal fixation part 50A in the axial direction of the guide wire 100A is smaller than the length L30 of the distal end side joint part 30 in the axial direction. Thus, in the guide wire 100A according to the second embodiment, the gap Ld between the distal end side joint part 30 and the metal fixation part 50A is relatively small. In other words, in the guide wire 100A, the metal fixation part 50A is located in the vicinity of the distal end of the guide wire 100A. Thus, in the guide wire 100A according to the second embodiment, the joining strength between the coil body 20 and the core shaft 10 can be more effectively ensured on the distal end of the guide wire 100A.

### C. Third Embodiment:

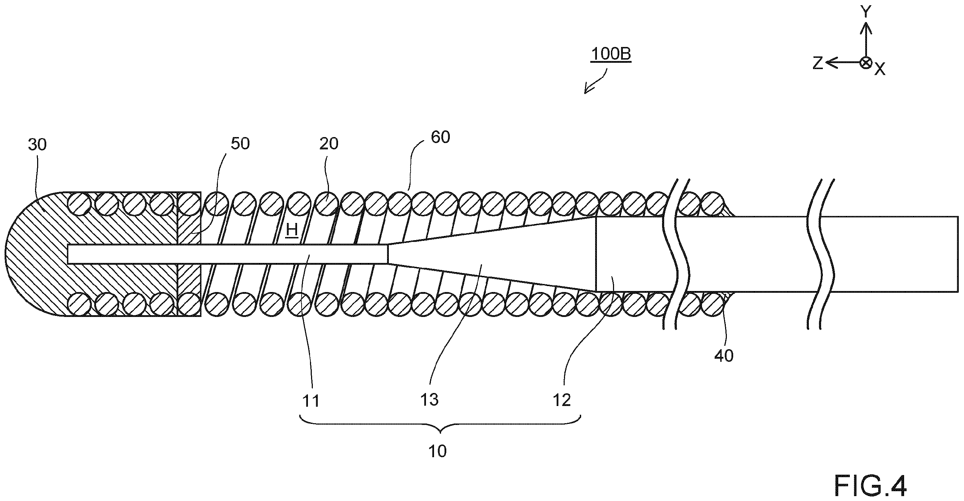

FIG. 4 is a schematic explanatory diagram illustrating a longitudinal sectional configuration (YZ section) of a guide wire 100B according to the third embodiment. Hereinafter, components of the guide wire 100B according to the third embodiment, which are identical to the components of the guide wire 100 according to the first embodiment, are provided with the same reference signs as in the first embodiment, thereby omitting the description thereof as appropriate.

The guide wire 100B according to the third embodiment does not have the resin part 60 in the guide wire 100 according to the first embodiment. The guide wire 100B according to the third embodiment exhibits effects excluding the effect of the resin part 60 among the effects in the guide wire 100 according to the first embodiment.

### D. Modifications:

The technology disclosed herein is not limited to the above-described embodiments and can be modified in various forms without departing from the scope of the present invention. For example, the following modifications are possible.

Although the configuration in which the outer surface of the coil body 20 is embedded in the metal fixation part 50 is adopted in the first embodiment, the present invention is not limited to this configuration. That means, it is possible to adopt a configuration in which the outer surface of the coil body 20 is in contact with the resin part 60 on a portion where the metal fixation part 50 is disposed such that a part of the outer surface of the coil body 20 is not embedded in the metal fixation part 50. The same applies to the second embodiment and the third embodiment. In the third embodiment, it is possible to adopt a configuration in which the outer surface of the coil body 20 is exposed.

Although the configuration in which the end face S30 of the distal end side joint part 30 and the end face S50 of the metal fixation part 50 are in contact with each other over the entire face is adopted in the first embodiment, the present invention is not limited to this configuration. That means, it is possible to adopt a configuration in which the end face S30 and the end face S50 are not in contact with each other at least in a part.

In the second embodiment, the gap Ld between the distal end side joint part 30 and the metal fixation part 50 may be equal to or larger than the length L30 of the distal end side joint part 30.

In the above embodiments, the length L30 of the distal end side joint part 30 may be less than twice and more than 10 times the outer diameter D20 of the coil body 20.

In the first and second embodiments, the resin part 60 may be configured to cover at least a part of each outer surface of the distal end side joint part 30 and the metal fixation part 50. In the guide wires 100 and 100A having the resin part 60, the distal end side joint part 30 may be made of a resin material different from the resin material for the resin part 60.

The material for each member according to the above embodiments is merely an example and may be variously modified. Also, the production method for the guide wires according to the above embodiments is merely an example and may be variously modified. For example, although the metal fixation part 50 is formed after the distal end side joint part 30 is formed in the above embodiments, the distal end side joint part 30 may be formed after the metal fixation part 50 is formed. In the above embodiments, although the most distal end portion 33 of the distal end side joint part 30 is configured to have an almost spherical segment shape, the present invention is not limited to this configuration, and the most distal end portion 33 may have another shape.

### DESCRIPTION OF REFERENCE NUMERALS

10: Core shaft
11: Small diameter portion
12: Large diameter portion
13: Tapered portion
20: Coil body
30: Distal end side joint part
31: Fixing portion
33: Most distal end portion
40: Proximal end side joint part
50: Metal fixation part
50A: Metal fixation part
60: Resin part
100: Guide wire
100A: Guide wire
100B: Guide wire
D20: Outer diameter
H: Lumen
L30: Length
L31: Length
L33: Length
L50: Length
Ld: Gap
S30: End face
S50: End face

## Claims

1. A guide wire comprising:
a core shaft;
a coil body that covers an outer periphery of the core shaft; and
a distal end side joint part that joins a distal end portion of the coil body with a distal end portion of the core shaft,
wherein the distal end side joint part is made of a resin material, and
the guide wire further comprises a fixation part made of a metal material that joins the coil body with the core shaft on a proximal end side of the distal end side joint part.

2. The guide wire according to claim 1, wherein the distal end side joint part is made of an elastomer material.

3. The guide wire according to claim 1 or 2, wherein a gap between the distal end side joint part and the fixation part in an axial direction of the guide wire is smaller than a length of the distal end side joint part in the axial direction.

4. The guide wire according to claim 1 or 2, wherein the distal end side joint part and the fixation part are continuously arranged in an axial direction of the guide wire.

5. The guide wire according to any one of claims 1 to 4, wherein a length of the distal end side joint part in an axial direction of the guide wire is twice or larger and 10 times or smaller an outer diameter of the coil body.

6. The guide wire according to any one of claims 1 to 5, wherein the guide wire further comprises a resin film that covers an outer surface of the distal end side joint part and an outer peripheral surface of the coil body and is made of one or a plurality of resin materials, and
the resin material for the distal end side joint part is the same as at least one of the resin materials for the resin film.
